# EUROPEAN PATENT APPLICATION

(11) **EP 4 454 772 A1**
(43) Date of publication of application: **30.10.2024**
(21) Application number: 24172051.5
(22) Date of filing: 24.04.2024
(51) Int. Cl.: B09B 3/45, A61L 2/07, A61L 11/00, B29B 17/02, B01D 21/26, B09B 101/67, B29L 31/48

(54) **METHOD FOR PROCESSING A WASTE STREAM CONTAINING SUPERABSORBENT POLYMERS (SAP)**

(30) Priority: 24.04.2023 NL 2034672
(71) Applicant: Elsinga Beleidsplanning en Innovatie B.V., 6708 WH Wageningen (NL)
(72) Inventor: ELSINGA, Willem, Wageningen (NL); MORSSINKHOF, Roy Hendrikus Norbertus, Wageningen (NL); KUIK, Job Gerardus Jacobus, Wageningen (NL)
(74) Representative: Algemeen Octrooi- en Merkenbureau B.V.

(57) **Abstract**

The present invention relates to a method for processing a waste stream containing superabsorbent polymers (SAP), comprising one or more of diapers, feminine hygiene products and incontinence materials, wherein superabsorbent polymers are incorporated into the above-mentioned products, wherein the above-mentioned waste stream is introduced into an autoclave and treated therein under high pressure and temperature to obtain one or more residual streams.

## Description

The present invention relates to a method for processing a waste stream containing superabsorbent polymers (SAP), comprising one or more of diapers, feminine hygiene products and incontinence materials, wherein superabsorbent polymers are incorporated into the above-mentioned products, and wherein the above-mentioned waste stream is introduced into an autoclave and treated therein under high pressure and temperature to obtain one or more residual streams.

The term SAP stands for superabsorbent polymers. Superabsorbent indicates that SAPs have a high capacity to absorb moisture. A superabsorbent polymer can absorb and retain up to 300 times its own weight in moisture. SAPs are usually contained as powder or granules in materials in which moisture needs to be absorbed. Examples of such materials are mainly personal hygiene materials such as diapers, feminine hygiene products, including sanitary napkins and tampons, and incontinence materials for adults. As an SAP absorbs moisture, the powder or granule changes into a gelled material. SAPs often contain acrylic acid. The most commonly used SAP in sanitary napkins and diapers is polyacrylate. In addition to acrylic acid, an SAP can also be composed of acrylamide (polyacrylamide). Previously, an SAP could also be composed of alcohol (polyvinyl alcohol) or ethylene oxide (polyethylene oxide).

According to the report "Composition of Residual Household Waste, Sorting Analyses 2019-May 2020" of the Ministry of Infrastructure and Water Management, in 2018, incontinence material accounted for 8.3% of residual household waste. This includes moisture and faecal matter. Beginning in the late 1980s, the amount of incontinence material in residual household waste increased sharply. Beginning in this period, disposable diapers came into widespread use in the Netherlands. In the period from 1995 through 2000, the percentage was more than 10%. After this, the percentage decreased to less than 5% in 2005. This decrease is attributable partly to the fact that in this period, separate collection and processing of incontinence material was begun in parts of the Netherlands, and partly to changes in the composition of incontinence materials that made them lighter. In recent years, the percentage has again increased.

International application WO 2013/171248 discloses a method for separating the components of a cellulose-plastic mixed product, namely cellulose and plastic, wherein in an autoclave, by means of a thermal pressure hydrolysis process, a separated plastic-containing ball can be removed from the autoclave, and the remaining cellulose hydrolysate forms a fermentable substrate. The method known from the above-mentioned International application requires that water be added to the starting material, thus increasing the energy requirement of the process. Moreover, the end product obtained by the above method will contain a large amount of water, which is undesirable.

A thermal hydrolysis process is also disclosed in International application WO 2015/097254, said process providing a continuous method for the treatment of sludge, comprising at least: a. a step of destructuring the sludge; b. a step of thermal hydrolysis of the thus destructured sludge in a thermal hydrolysis reactor; and c. a step of cooling the hydrolysed sludge. According to WO 2015/097254, the destructuring step comprises a step of introducing the sludge to be treated into a dynamic mixer and then heating the sludge in the dynamic mixer.

European patent application EP 2919822 discloses a method for releasing water from swollen superabsorbent polymers (SAPs), said method comprising the immersion of a swollen SAP in a gelled state in a composition for removing moisture, wherein the composition for removing moisture comprises a mixture of seawater and 0.5 to 3 wt% of calcium chloride based on the weight of seawater, with the swollen SAP being immersed in a gelled state in the composition for removing moisture for 10 min to 4 h, wherein the seawater is either natural seawater that has not undergone any further processing or purification or artificial seawater.

The object of the present invention is to provide a method to be carried out in an autoclave for processing a starting material containing organic components, comprising one or more of diapers, feminine hygiene products and incontinence materials, wherein under application of high pressure and temperature, one or more residual streams are obtained that can be reused in a simple and useful manner.

Yet another object of the present invention is to provide a method to be carried out in an autoclave for processing a starting material containing organic components, comprising one or more of diapers, feminine hygiene products and incontinence materials, wherein the superabsorbent polymers originally present in the waste material can be recovered.

The present invention thus relates to a method for processing a waste stream containing superabsorbent polymers (SAP), comprising one or more of diapers, feminine hygiene products and incontinence materials, wherein superabsorbent polymers are incorporated into the above-mentioned products, wherein the above-mentioned waste stream is introduced into an autoclave and treated therein under the conditions of a temperature of at least 200°C, a pressure of at least 20 bar and a residence time of at least 0.1 h to obtain one or more residual streams, characterized in that after completion of the treatment, steam is released from the autoclave and condensed to obtain a condensate with a pH > 8 and an aqueous residual stream remaining in the autoclave with a pH < 6, wherein the conditions during the residence time of the waste stream in the autoclave are such that the superabsorbent polymers are converted into soluble compounds that are dissolved in the aqueous residual stream.

Specifically, the inventors of the present invention have found that when the superabsorbent polymers, which were originally present in the waste stream as solid matter or a gel, are converted in the autoclave into soluble compounds, the soluble compounds will be in an aqueous residual stream, and said aqueous residual stream can further be processed in order to meet one or more of the original objectives. The inventors of the present invention have found that according to the above-mentioned method, as known from International application WO 2013/171248, a ball of plastic is formed in the reactor that is difficult to remove from said reactor.

The condensate obtained in the present method is thus characterized by a basic pH, said basic value being attributable to the presence of basic components, in particular ammonium nitrogen. The aqueous residual stream remaining in the autoclave has a pH of < 6 and is characterized by the presence of a high chloride content, in particular > 1000 mg/l. The inventors of the present invention assume that the solubility of the superabsorbent polymers converted as soluble compounds is a somewhat reversible process, wherein it can be ensured for example by increasing the pH that precipitate formation of the superabsorbent polymers occurs. It is also assumed that the addition of certain ions, such as ferric chloride, decreases the solubility and leads to precipitate formation.

In an example of the present method, the aqueous residual stream obtained from the autoclave is first stripped of solid components using a sieving method, after which the residual stream with its solid components removed is then further treated. The application of a sieving method, which involves a filter, screw press or sieve, makes it possible for the solid components to be released, said solid components mainly comprising recyclable plastics, and they can be further processed, for example as plastic granules or energy carriers for an incineration process. The removal of the solid components is also advantageous because they cannot occur in the further course of the process as interfering factors.

In an example of the present method, the dry matter content of the above-mentioned aqueous residual stream with the solid components removed is increased, specifically by subjecting the aqueous residual stream with the solid components removed to one or more process steps selected from evaporation and drying. The dry matter content of the aqueous residual stream with the solid components removed is ordinarily in the range of 4-14 wt% and is increased by the above-mentioned after-treatment to a value of greater than 20 wt%, preferably greater than 25 wt%, and more preferably greater than 30 wt%. The favourable advantage of increasing the dry matter content is that this gives rise to a higher polymer content of the solution, which is thus better suited for application as a raw material for new products. In addition, this saves on logistical costs because a smaller amount of water is transported. An additional advantage of evaporating the aqueous residual stream with the solid components removed is that in addition to the evaporated polymer solution with a higher polymer content, a clean aqueous stream is also produced from the condensate obtained. This aqueous condensate stream is so clean that it requires no or only minimal additional purification and can be used as water for a variety of recycling purposes.

In an example of the present method, a precipitant is added to the aqueous residual stream with the solid components removed containing the above-mentioned soluble compounds, said precipitant forming a precipitate with the above-mentioned soluble compounds, after which the residual stream thus treated with the precipitant is separated into a stream having a high precipitate content and a stream having a high water content. By means of such a precipitation reaction, the soluble compounds generated in the autoclave by the superabsorbent polymers are removed from the aqueous residual stream with the solid components removed and concentrated in a separate stream, with said stream having a high precipitate content thus being suitable for further application. The amount of precipitant to be added is determined by the amount of soluble compounds, with the latter amount being determined by the amount of SAP contained in the autoclave. In particular, an amount of precipitant is added such that all of the soluble compounds are converted into a precipitate.

According to another example of the present method, the aqueous residual stream with the solid components removed is stripped of fibrous components in an additional step, wherein a stream having a high fibrous fraction content and a stream having a low fibrous fraction content are obtained, after which the stream having a low fibrous fraction content is then further treated. Centrifugation can be mentioned as an example of such a separating step. As a result of the above-mentioned additional step, the stream having a high fibrous fraction content will have a higher dry matter content than the stream having a low fibrous fraction content, and the latter stream can be described as aqueous. The stream having a low fibrous fraction content will mainly contain the superabsorbent polymers converted in the autoclave into soluble compounds.

In an example of the present method, the stream having a high fibrous fraction content is subjected to one or more process steps selected from fermentation, composting or drying. Actually, the superabsorbent polymers converted in the autoclave into soluble compounds should be contained mainly in the stream having a low fibrous fraction content, which makes the stream having a high fibrous fraction content suitable for being subjected to a biological process such as fermentation and composting. The inventors of the present invention have found that the superabsorbent polymers converted into soluble compounds are less well-suited for composting or fermentation.

In an example of the present method, the dry matter content of the above-mentioned stream having a low fibrous fraction content is increased, in particular by subjecting the above-mentioned stream having a low fibrous fraction content to one or more process steps selected from evaporation and drying.

In an example of the present method, a precipitant is added to the above-mentioned stream having a low fibrous fraction content containing the above-mentioned soluble compounds, said precipitant forming a precipitate with the above-mentioned soluble compounds, after which the residual stream thus treated with the precipitant is separated into a stream having a high precipitate content and a stream having a high water content. By means of such a precipitation reaction, the soluble compounds generated in the autoclave by the superabsorbent polymers are removed from the aqueous residual stream with the solid components and concentrated in a separate stream, said stream having a high precipitate content and thus being suitable for further application. The amount of precipitant to be added is determined by the amount of soluble compounds, with the latter amount being determined by the amount of SAP contained in the autoclave. In particular, an amount of precipitant is added such that all of the soluble compounds are converted into a precipitate.

In an example of the present method, the stream having a high water content is supplied to a post-treatment facility selected from the group of a sewage treatment plant and a fermentation plant.

In an example of the present method, the stream having a high precipitate content is further processed as a raw material for polymer-containing products such as dyes and asphalt, fuel for an incineration plant, aggregate for concrete and/or filling material.

In an example of the present method, the precipitant is selected from one or more agents from the group of calcium chloride, calcium acetate, methanol, potassium hydroxide, ferric chloride and sodium chloride, preferably calcium chloride. The inventors of the present invention assume that polyacrylate molecules are converted by undissolved water-binding gelled crosslinked polyacrylate molecules to a dissolved form by more or less the same polyacrylate molecules, wherein the crosslinks are broken. The resulting precipitates can be collected by filtration.

In an example of the present method, the above-mentioned waste stream to be treated in the autoclave further comprises one or more additional components selected from the group of oils, fats, sludge, in particular sludge from a waste water treatment plant, vegetable, food, and garden waste digestate and emulsifiers. Although the present description mentions diapers, feminine hygiene products and incontinence materials, it is to be clearly understood that the waste stream to be treated can also comprise other unavoidable components. Examples in this context include tissues, wet toilet paper, baby wipes, sanitary napkins, toilet paper and the like. In many municipalities, special bags are distributed for the collection of such waste materials. Diapers and incontinence materials are thus collected separately by means of special collection bags that usually can be obtained free of charge. The separate collection provides the municipality with the possibility of recycling such materials. It is also possible for the diapers to be separated from the stream of residual household waste in a post-separation plant and then applied in this separated form as raw materials to be further processed in the present method.

The conditions during the residence of the waste stream in the autoclave comprise a temperature of at least 200°C, a pressure of at least 20 bar and a residence time of at least 0.1 h. Preferably, during residence in the autoclave, the contents thereof should be kept in motion, in particular by means of an agitator positioned in the autoclave. The inventors of the present invention assume that the application of process conditions that deviate from those specified herein will lead to at least one of the following results, specifically insufficient hydrolysis of the organic components, incomplete melting of the plastics, and incomplete dissolution of the SAPs. This makes it impossible to achieve favourable separation among the various streams and the SAPs. The assumption is that in this case, the SAPs will still be partially present in gel form and will be insufficiently separated from the other solid components.

The present invention will now be explained by means of several examples, but it should be noted that the present invention is by no means limited by such particular examples.
Fig. 1 shows a first embodiment of the present invention for processing a waste stream containing superabsorbent polymers (SAP).
Fig. 2 shows a second embodiment of the present invention for processing a waste stream containing superabsorbent polymers (SAP).

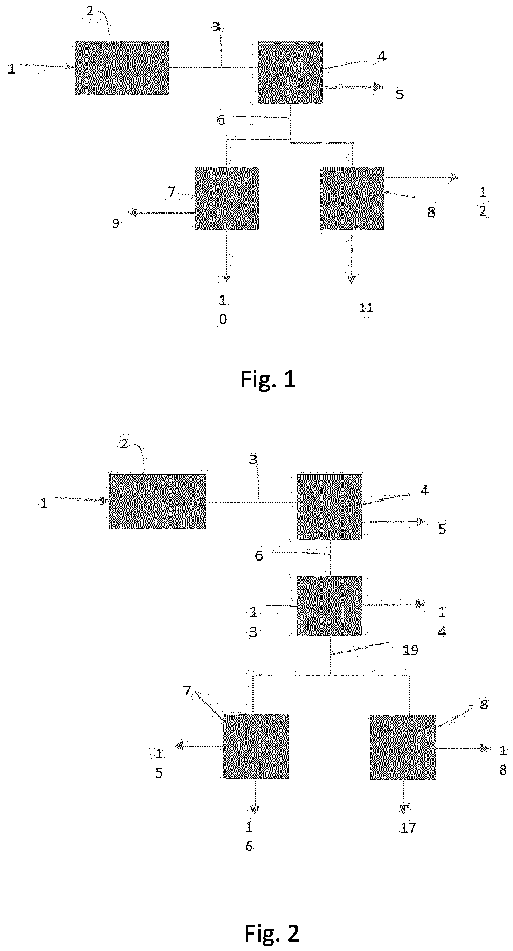

In Fig. 1, a waste stream 1 comprising one or more of diapers, feminine hygiene products and incontinence materials is supplied to an autoclave 2. After residence in the autoclave 2 under high pressure and temperature, one or more residual streams 3 are obtained. Residual stream 3 is separated in unit 4, for example a screening plant, into a stream 5 containing solid components and an aqueous residual stream 6 with the solid components removed. Residual stream 6 is subjected to further treatment selected from a unit 7 for increasing the dry matter content and a unit 8 in which precipitation takes place. In unit 7 for increasing the dry matter content, a process step selected from evaporation and drying is carried out, resulting in an aqueous residual stream 9 and a residual stream 10, the dry matter content of which is higher than that of the stream originally supplied to unit 7, i.e. the aqueous residual stream 6 with the solid components removed. In unit 8, a precipitation reaction is carried out by adding a precipitant to the aqueous residual stream 6 with the solid components removed, resulting in a stream 11 having a high precipitate content and a stream 12 having a high water content.

Although the process diagram shown in Fig. 1 shows that the aqueous residual stream 6 is supplied to both units 7 and 8, it is possible in practice for the entire aqueous residual stream 6 to be supplied to only one of the units 7 and 8, e.g., the aqueous residual stream 6 is completely treated with a precipitant in unit 8.

The process diagram shown in Fig. 2 coincides with a number of steps of the process diagram shown in Fig. 1. Waste stream 1, comprising one or more of diapers, feminine hygiene products and incontinence materials, is supplied to an autoclave 2. After residence in autoclave 2 under high pressure and temperature, one or more residual streams 3 is/are obtained. Residual stream 3 is separated in unit 4, for example a screening plant, into a stream 5 containing solid components and an aqueous residual stream 6 with the solid components removed. The aqueous residual stream 6 with the solid components removed is supplied to a separating unit 13, such as a centrifuge, in order to carry out separation of the fibrous fraction therein. In separating unit 13, a stream 14 with a high fibrous fraction content and a stream 19 with a low fibrous fraction are thus obtained, after which stream 19 with a low fibrous fraction is further treated. This further treatment comprises a unit 7 for increasing the dry matter content and/or a unit 8 in which precipitation takes place. In unit 7 for increasing the dry matter content, a process step selected from evaporation and drying is carried out, resulting in an aqueous residual stream 15 and a residual stream 16 having a higher dry matter content than that of the stream originally supplied to unit 7, i.e. the stream 19 with a low fibrous fraction content exiting unit 13. In unit 8, a precipitation reaction is carried out by adding a precipitant to the stream 19 with a low fibrous fraction content exiting unit 13, resulting in a stream 17 having a high precipitate content and a stream 18 having a high water content.

It also applies for Fig. 2 that although the process diagram shown in Fig. 2 shows that the stream 19 with a low fibrous fraction content is supplied to both units 7 and 8, it is possible in practice for the entire stream 19 with a low fibrous fraction content to be supplied to only one of the units 7 and 8, e.g. stream 19 with a low fibrous fraction content is completely treated with a precipitant in unit 8.

## Claims

1. Method for processing a waste stream containing superabsorbent polymers (SAPs), comprising one or more of diapers, feminine hygiene products and incontinence materials, wherein superabsorbent polymers are incorporated into the above-mentioned products, wherein the above-mentioned waste stream is introduced into an autoclave and treated therein under the conditions of a temperature of at least 200°C, a pressure of at least 20 bar and a residence time of at least 0.1 h to obtain one or more residual streams, **characterised in that** after completion of the treatment, steam is released from the autoclave and condensed to obtain a condensate with a pH > 8 and an aqueous residual stream remaining in the autoclave with a pH < 6, wherein the conditions during the residence time of the waste stream in the autoclave are such that the superabsorbent polymers are converted into soluble compounds that are dissolved in the aqueous residual stream.

2. Method according to one or more of the preceding claims, **characterised in that** the aqueous residual stream obtained from the autoclave first has its solid components removed by means of a sieving method, after which the residual stream with its solid components removed is then further treated.

3. Method according to Claim 2, **characterised in that** the dry matter content of the above-mentioned aqueous residual stream with the solid components removed is increased, in particular by subjecting the aqueous residual stream with the solid components removed to one or more process steps selected from evaporation and drying.

4. Method according to one or more of the preceding claims, **characterised in that** a precipitant is added to the aqueous residual stream with the solid components removed containing the above-mentioned soluble compounds, the precipitant forming a precipitate with the above-mentioned soluble compounds, after which the residual stream thus treated with the precipitant is separated into a stream having a high precipitate content and a stream having a high water content.

5. Method according to Claim 2, **characterised in that** in an additional step, the aqueous residual stream with the solid components removed is stripped of fibrous components, wherein a stream having a high fibrous fraction content and a stream having a low fibrous fraction content are obtained, after which the stream having a low fibrous fraction content is then further treated.

6. Method according to Claim 5, **characterised in that** the stream having a high fibrous fraction content is subjected to one or more process steps selected from fermentation, composting or drying.

7. Method according to Claim 5, **characterised in that** the dry matter content of the above-mentioned stream having a low fibrous fraction content is increased, in particular by subjecting the above-mentioned stream having a low fibrous fraction content to one or more process steps selected from evaporation and drying.

8. Method according to one or more of Claims 5-7, **characterised in that** a precipitant is added to the above-mentioned stream having a low fibrous fraction content containing the above-mentioned soluble compounds, said precipitant forming a precipitate with the above-mentioned soluble compounds, after which the residual stream thus treated with the precipitant is separated into a stream having a high precipitate content and a stream having a high water content.

9. Method according to one or more of Claims 4-8, **characterised in that** the stream having a high water content is supplied to a post-treatment facility selected from the group of a sewage treatment plant and a fermentation plant.

10. Method according to one or more of Claims 4-9, **characterised in that** the stream having a high precipitate content is further processed as a raw material for polymer-containing products such as dyes and asphalt, fuel for an incineration plant, aggregate for concrete and/or a filling material.

11. Method according to one or more of Claims 4-10, **characterised in that** the precipitant is selected from one or more agents from the group of calcium chloride, calcium acetate, methanol, potassium hydroxide, ferric chloride and sodium chloride, preferably calcium chloride.

12. Method according to one or more of the preceding claims, **characterised in that** the above-mentioned waste stream to be treated in the autoclave further comprises one or more additional components selected from the group of oils, fats, sludge, in particular sludge from a waste water treatment plant, vegetable, food, and garden waste digestate and emulsifiers.

13. Method according to one or more of the preceding claims, **characterised in that**.
